# EUROPEAN PATENT APPLICATION

(11) **EP 3 750 577 A1**
(43) Date of publication of application: **16.12.2020**
(21) Application number: 19750340.2
(22) Date of filing: 08.02.2019
(51) Int. Cl.: A61M 5/30, A61K 31/713, A61K 45/00, A61K 48/00, A61M 5/303, A61P 43/00, A61K 49/00

(54) **INJECTOR AND METHOD OF INJECTING SOLUTION CONTAINING LIVE CELLS INTO INJECTION-TARGET CELL NUCLEI USING SAID INJECTOR**

(30) Priority: 09.02.2018 JP 2018021910
(71) Applicant: Daicel Corporation, Osaka 530-0011 (JP)
(72) Inventor: MIKI, Katsuya, Tokyo 108-8230 (JP); ATOBE, Shingo, Tokyo 108-8230 (JP); MIYAZAKI, Hiroshi, Tokyo 108-8230 (JP); SUZUKI, Ayano, Tokyo 108-8230 (JP); SAKAGUCHI, Yuko, Tokyo 108-8230 (JP)
(74) Representative: Grünecker Patent- und Rechtsanwälte PartG mbB
(86) International application number: PCT/JP2019/004724
(87) International publication number: WO 2019/156239

(57) **Abstract**

An object of the present invention is to provide an injector that can directly inject a solution containing biomolecules into a cell nucleus of an injection target with high efficiency and a method of directly injecting a solution containing biomolecules into a cell nucleus of an injection target using the injector with high efficiency; and the object is fulfilled by an injector that injects a solution containing biomolecules into an injection target from an injector main body without performing injection through a given structure in a state where the given structure is inserted into the injection target, the injector comprising: an accommodation unit for accommodating a solution containing biomolecules; and a nozzle unit including an injection port through which the solution containing biomolecules flows and is injected into the injection target, the solution being pressurized, wherein there is a time at which an injection speed of the solution containing biomolecules is 40 m/s or more between an injection start time of the solution containing biomolecules and a time of 0.20 ms.

## Description

### TECHNICAL FIELD

The present invention relates to an injector and a method of injecting a solution containing biomolecules into a cell nucleus of an injection target using the same.

### BACKGROUND ART

Regarding injectors for injecting a drug solution into a living body or the like, there are catheters including an injection needle and a drive source for transporting a drug solution into an injection target in addition to a needle syringe that performs injection through an injection needle and a needleless syringe that performs injection without using an injection needle.

Among these, a needleless syringe may be configured to inject an injection component by applying a pressure to an accommodation chamber in which an injection solution is accommodated using a pressurized gas, a spring, or an electromagnetic force. For example, a configuration in which a plurality of nozzle holes are formed inside a syringe main body and a piston that is driven during injection is arranged to correspond to each nozzle hole may be used (Patent Document 1). With such a configuration, an injection solution is sprayed simultaneously from a plurality of nozzle holes and uniform injection into a target is realized. Then, a plasmid containing a luciferase gene can be injected into rats and cells can be transferred with high efficiency.

In addition, there is a form in which a pressurized gas is used as an injection power source for an injection solution in a needleless syringe. For example, a pressurization form in which a high pressure is instantaneously applied in the initial stage of injection, and the applied pressure is then gradually reduced over 40 to 50 msec may be exemplified (Patent Document 2).

However, there are no reports in which a solution containing biomolecules can be directly injected into a cell nucleus of an injection target by an injector with high efficiency. In addition, there are no reports focusing on conditions for injecting a solution containing biomolecules from an injector required for directly injecting a solution containing biomolecules into a cell nucleus of the injection target with high efficiency.

### PRIOR ART DOCUMENTS

### Patent Document

[Patent Document 1] Japanese Patent Application Publication No. 2004-358234
[Patent Document 2] U.S. Patent Application Publication No. 2005/0010168

### SUMMARY OF THE INVENTION

### PROBLEMS TO BE SOLVED BY THE INVENTION

The present invention has been made in view of such circumstances, and an object of the present invention is to provide an injector that can directly inject a solution containing biomolecules into a cell nucleus of an injection target with high efficiency and a method of directly injecting a solution containing biomolecules into a cell nucleus of an injection target using the injector with high efficiency.

### MEANS FOR SOLVING THE PROBLEMS

The inventors conducted extensive studies and as a result, found that, in an injector in which a solution containing biomolecules is accommodated, by focusing on an injection speed of the solution containing biomolecules within a predetermined time from an injection start time of the solution containing biomolecules injected by the injector, the following injector can address the above problems, and thus completed the present invention. The present invention is as follows.
[1] An injector that injects a solution containing biomolecules into an injection target from an injector main body without performing injection through a given structure in a state where the given structure is inserted into the injection target, the injector comprising:
   an accommodation unit for accommodating a solution containing biomolecules; and
   a nozzle unit including an injection port through which the solution containing biomolecules flows and is injected into the injection target, the solution being pressurized,
   wherein there is a time at which an injection speed of the solution containing biomolecules is 40 m/s or more between an injection start time of the solution containing biomolecules and a time of 0.20 ms.
[2] The injector according to [1],
   wherein there is a time at which the injection speed of the solution containing biomolecules is 75 m/s or more between the injection start time of the solution containing biomolecules and a time of 0.20 ms.
[3] The injector according to [1] or [2],
   wherein there is a time at which the injection speed of the solution containing biomolecules is 75 m/s or more between the injection start time of the solution containing biomolecules and a time of 0.15 ms.
[4] A method of injecting a solution containing biomolecules into a cell nucleus of an injection target using the injector according to any one of [1] to [3].

### EFFECT OF THE INVENTION

According to the present invention, it is possible to provide an injector that can directly inject a solution containing biomolecules into a cell nucleus of an injection target with high efficiency and a method of directly injecting a solution containing biomolecules into a cell nucleus of an injection target using the injector with high efficiency.

### BRIEF DESCRIPTION OF THE DRAWINGS

Fig. 1 is a diagram showing a schematic configuration of an injector according to one embodiment of a first aspect of the present invention.
Fig. 2 is a graph showing an injection speed of filled water over time according to one embodiment of the first aspect of the present invention.
Fig. 3-1 is a diagram (photograph annotated with drawing) showing a distribution of DNA injected into a cell nucleus in a mammalian individual (living body) and a mammalian individual (living body) according to one embodiment of a second aspect of the present invention.
Fig. 3-2 is a diagram (photograph annotated with drawing) showing a distribution of DNA injected into a cell nucleus in a mammalian individual (living body) and a mammalian individual (living body) according to one embodiment of a second aspect of the present invention.
Fig. 3-3 is a diagram (photograph annotated with drawing) showing a distribution of DNA injected into a cell nucleus in a mammalian individual (living body) and a mammalian individual (living body) according to one embodiment of a second aspect of the present invention.

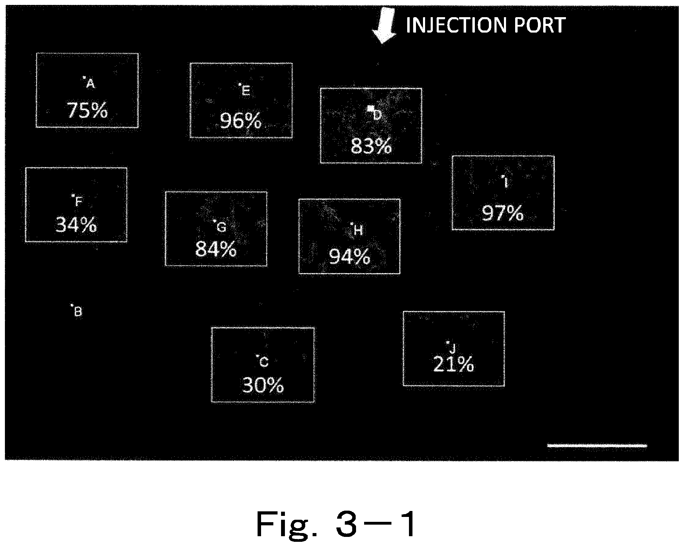

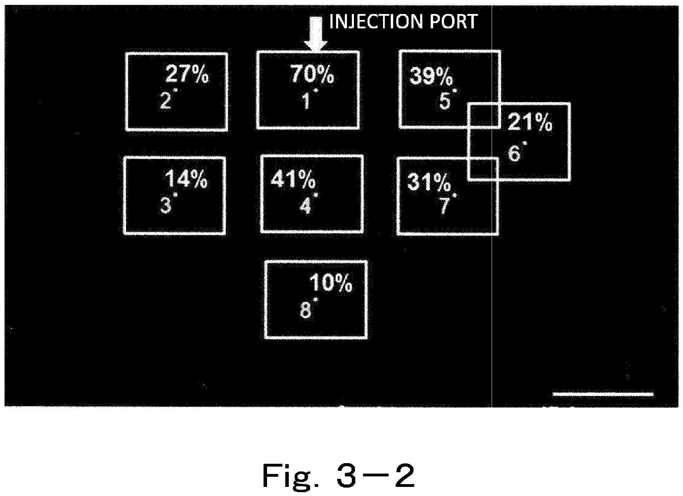

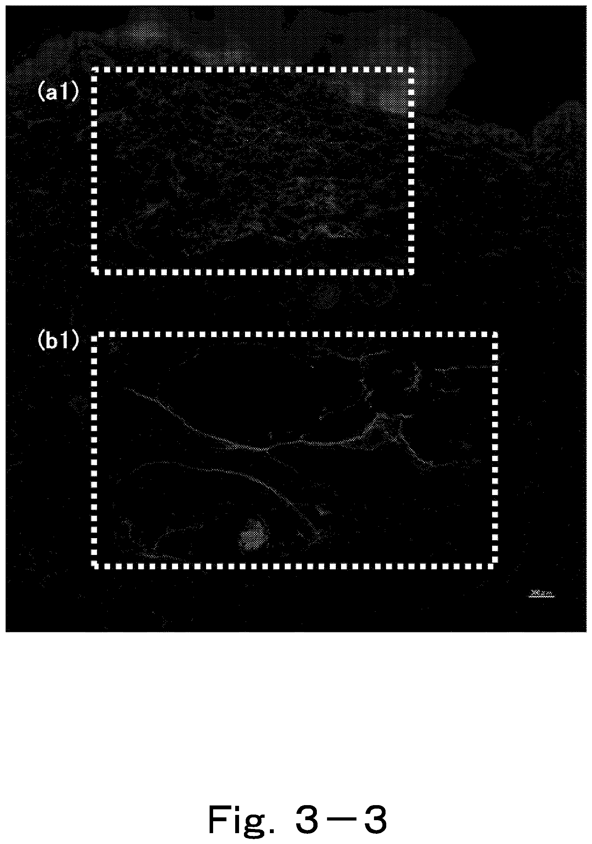

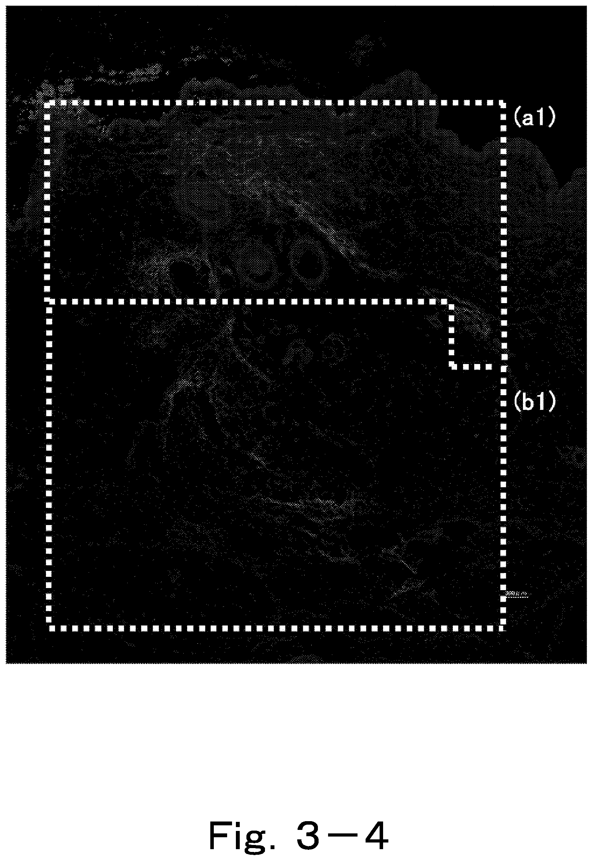

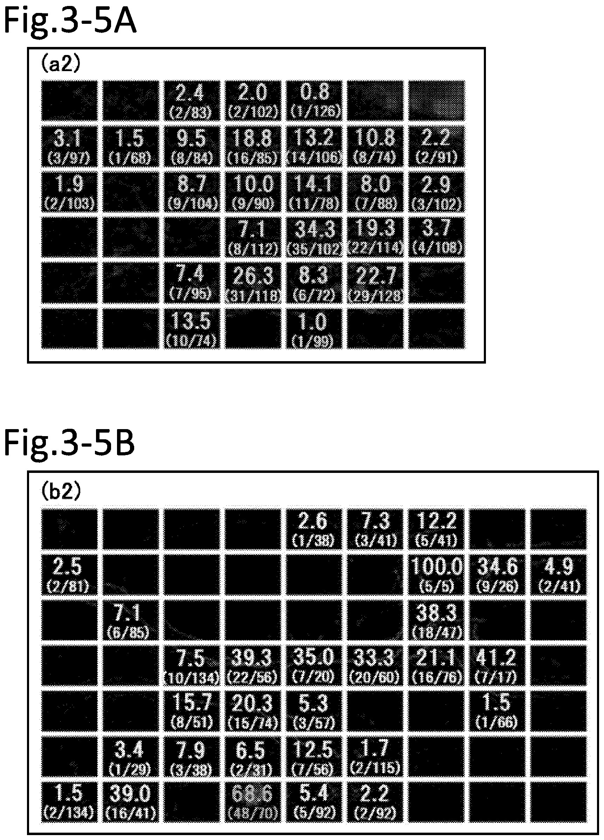

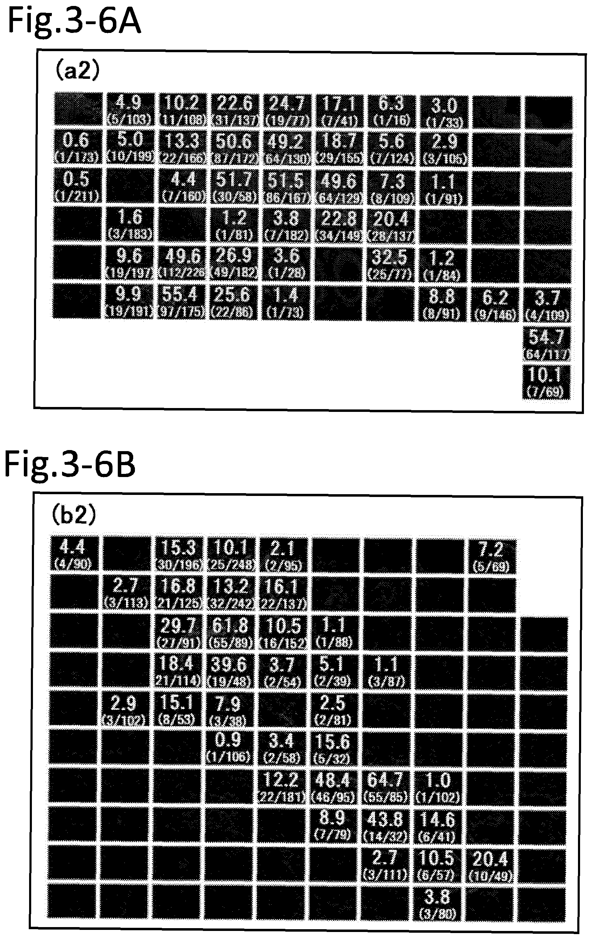

Fig. 3-4 is a diagram (photograph annotated with drawing) showing a distribution of DNA injected into a cell nucleus in a mammalian individual (living body) and a mammalian individual (living body) according to one embodiment of a second aspect of the present invention.
Fig. 3-5A is a diagram (photograph annotated with drawing) showing a distribution of a proportion of the number of cells into which DNA has been directly injected into a cell nucleus according to one embodiment of the second aspect of the present invention.
Fig. 3-5B is a diagram (photograph annotated with drawing) showing a distribution of a proportion of the number of cells into which DNA has been directly injected into a cell nucleus according to one embodiment of the second aspect of the present invention.
Fig. 3-6A is a diagram (photograph annotated with drawing) showing a distribution of a proportion of the number of cells into which DNA has been directly injected into a cell nucleus according to one embodiment of the second aspect of the present invention.
Fig. 3-6B is a diagram (photograph annotated with drawing) showing a distribution of a proportion of the number of cells into which DNA has been directly injected into a cell nucleus according to one embodiment of the second aspect of the present invention.

### MODE FOR CARRYING OUT THE INVENTION

The present invention includes an invention of an injector (first aspect) and an invention of a method of injecting a solution containing biomolecules to a cell nucleus of an injection target using the injector (second aspect).

### <First aspect>

The first aspect of the present invention is an injector that injects a solution containing biomolecules into an injection target from an injector main body without performing injection through a given structure in a state where the given structure is inserted into the injection target, the injector comprising: an accommodation unit for accommodating a solution containing biomolecules; and a nozzle unit including an injection port through which the solution containing biomolecules flows and is injected into the injection target, the solution being pressurized, wherein there is a time at which an injection speed of the solution containing biomolecules is 40 m/s or more between an injection start time of the solution containing biomolecules and a time of 0.20 ms.

In the injector according to the first aspect of the present invention, since there is a time at which the injection speed of the solution containing biomolecules is 40 m/s or more between the injection start time of the solution containing biomolecules and a time of 0.20 ms, it is possible to directly inject the solution containing biomolecules into a cell nucleus of the injection target with high efficiency.

Specifically, for example, when the injection target is cells in a mammalian individual (living body), there is a time at which the injection speed of the solution containing biomolecules is 40 m/s or more between the injection start time of the solution containing biomolecules and a time of 0.20 ms, and thus the solution containing biomolecules penetrates through the epidermis of the mammalian individual (living body), the cells are deformed due to a shear force when the solution is injected into the dermis, and the solution containing biomolecules is expected to be directly injected into the cell nucleus of the cells in the mammalian individual (living body) with high efficiency.

In this case, since the solution containing biomolecules is directly injected into a cell nucleus of the injection target with higher efficiency, preferably, there is a time at which the injection speed of the solution containing biomolecules is 75 m/s or more between the injection start time of the solution containing biomolecules and a time of 0.20 ms, and more preferably there is a time at which the injection speed of the solution containing biomolecules is 75 m/s or more between the injection start time of the solution containing biomolecules and a time of 0.15 ms. In addition, for example, when the injection target is cells in a mammalian individual (living body), since the solution containing biomolecules is expected to be injected into the injection target without penetrating through the mammalian individual (living body) itself, the injection speed of the solution containing biomolecules is preferably 250 m/s or less and more preferably 200 m/s or less between the injection start time of the solution containing biomolecules and a time of 0.20 ms. For example, when the injection target is cells in swine, and as a preferable form, when the injection target is cells in the skin of the abdomen, the injection speed is preferably 200 m/s or less. In addition, when the injection target is cells in a rat, and as a preferable form, when the injection target is cells in the skin of the lumbar back, the injection speed is preferably 150 m/s or less.

In the first aspect of the present invention, biomolecules injected into the cell nucleus of the injection target are not particularly limited as long as they function in the cell nucleus or cells of the injection target when they are injected into the cell nucleus of the injection target. In addition, the biomolecules may be a natural product or artificially synthesized product. Examples thereof include nucleic acids or derivatives thereof; nucleosides, nucleotides or derivatives thereof; amino acids, peptides, proteins or derivatives thereof; lipids or derivatives thereof; metal ions; low-molecular-weight compounds or derivatives thereof; antibiotics; and vitamins or derivatives thereof. The nucleic acid may be DNA or RNA, and may include a gene. In examples to be described below, a free Cy3-labeled plasmid DNA is used as biomolecules.

The form of the biomolecules to be injected into the cell nucleus of the injection target and a solvent therefor are not particularly limited as long as biomolecules are stably present and there is no adverse effect such as destruction of the injection target or the cell nucleus of the injection target to be injected, and may be a free form, a form in which biomolecules are fixed to carriers such as nanoparticles, a modified form.

When DNA contains a gene, a design form in which the gene is contained in an expression cassette or expression vector may be exemplified. In addition, for example, the gene may be provided under control of a promoter suitable for the type of the injection target into which the DNA is injected and the injection site. That is, in any of the forms, a known genetic engineering technique can be used.

In the injector according to the first aspect of the present invention, "distal end side" refers to the side on which an injection port through which a solution containing biomolecules is injected from an injector is arranged, and "proximal end side" refers to the side opposite to the distal end side in the injector, and these terms do not limit specific locations or positions.

The injector according to the first aspect of the present invention injects a solution containing biomolecules to the injection target from an injector main body without performing injection through a given structure in the state where the given structure is inserted into the injection target. The injector according to the first aspect of the present invention may have, for example, a given structure such as a catheter for guiding a solution containing biomolecules from an injector main body to an injection target, for example, when a distance from the injector main body to the injection target is large. Therefore, the injector according to the first aspect of the present invention may or may not have such a given structure. However, when the injector has such a given structure, a solution containing biomolecules is not injected into the injection target in the state where the given structure is inserted into the injection target.

In the injector according to the first aspect of the present invention, a driving unit for pressurizing a solution containing biomolecules is not particularly limited. The pressurization may be caused by, for example, a pressure generated when the pressure of the compressed gas is released, or a pressure generated by combustion of an explosive that is ignited by an ignition device. In addition, pressurization using an electromagnetic force, for example, pressurization using a linear electromagnetic actuator, may be used. Preferably, at least, a form in which a pressure generated by combustion of an explosive that is ignited by an ignition device is used, or any one of two other pressurization forms or a combination of them may be used.

When a form in which a pressure generated by combustion of an explosive that is ignited by an ignition device is used for pressurization is used, the explosive may be, for example, any explosive among an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), and an explosive containing aluminum and iron oxide (AFO) or an explosive composed of a plurality of combinations of these. Regarding a feature of these explosives, if the combustion products are gases in a high temperature state, since they do not contain gas components at room temperature, the combustion products after ignition immediately condense.

In addition, when the generated energy of a gas generating agent is used as injection energy, various gas generating agents used in a single base smokeless explosive, a gas generator for an airbag, and a gas generator for a seat belt pretensioner can be used as the gas generating agent.

In the injector according to the first aspect of the present invention, the solution containing biomolecules is not accommodated in a filling chamber from the beginning, and the solution containing biomolecules is accommodated in the filling chamber by sucking through a nozzle having an injection port. In this manner, when a configuration in which a filling operation in the filling chamber is required is used, it is possible to inject any required solution containing biomolecules into the injection target. Therefore, in the injector according to the first aspect of the present invention, a syringe part is removable.

Hereinafter, regarding an example of an injector according to one embodiment of the first aspect of the present invention, a syringe 1 (needleless syringe) will be described with reference to the drawings. Here, the configuration of the following embodiment is an example, and the first aspect of the present invention is not limited to the configuration of the embodiment. Here, the terms "distal end side" and "proximal end side" are used as terms that represent the relative positional relationships in the syringe 1 in the longitudinal direction. The "distal end side" represents a position near the tip of the syringe 1 to be described below, that is, near an injection port 31a, and the "proximal end side" represents a side on the side opposite to the "distal end side" of the syringe 1 in the longitudinal direction, that is, a side on the side of a driving unit 7. In addition, this example is an example in which combustion energy of an explosive that is ignited by an ignition device is used as injection energy and a DNA solution is used as a solution containing biomolecules, but the first aspect of the present invention is not limited thereto.

### (Configuration of syringe 1)

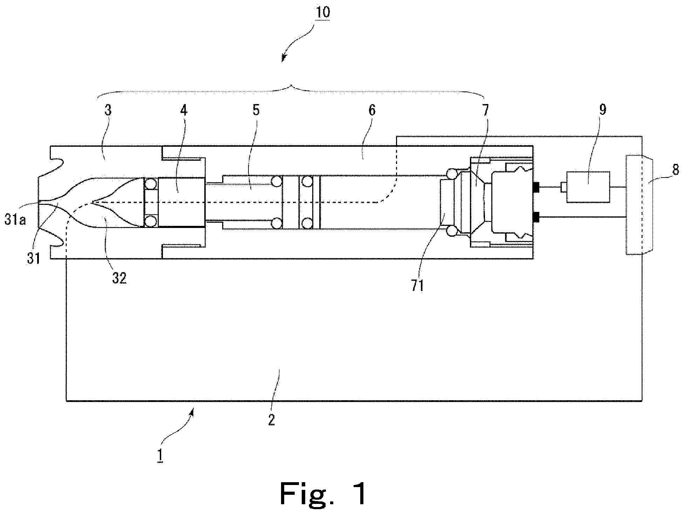

Fig. 1 is a diagram showing a schematic configuration of the syringe 1 and is a cross-sectional view of the syringe 1 in the longitudinal direction. The syringe 1 has a configuration in which a syringe assembly 10 in which a sub-assembly including a syringe part 3 and a plunger 4 and a sub-assembly including a syringe main body 6, a piston 5, and the driving unit 7 are integrally assembled is mounted in a housing (syringe housing) 2.

As described above, the syringe assembly 10 is configured to be detachable from the housing 2. A filling chamber 32 formed between the syringe part 3 and the plunger 4 included in the syringe assembly 10 is filled with a DNA solution, and the syringe assembly 10 is a unit that is discarded whenever the DNA solution is injected. On the other hand, on the side of the housing 2, a battery 9 that supplies power to an igniter 71 included in the driving unit 7 of the syringe assembly 10 is included. When a user performs an operation of pressing a button 8 provided in the housing 2, supply of power from the battery 9 is performed between an electrode on the side of the housing 2 and an electrode on the side of the driving unit 7 of the syringe assembly 10 via a wiring. Here, the shape and position of both electrodes are designed so that the electrode on the side of the housing 2 and the electrode on the side of the driving unit 7 of the syringe assembly 10 are automatically brought in contact when the syringe assembly 10 is mounted in the housing 2. In addition, the housing 2 is a unit that can be repeatedly used as long as power that can be supplied to the driving unit 7 remains in the battery 9. Here, in the housing 2, when the battery 9 has no power, only the battery 9 may be replaced, and the housing 2 may be continuously used.

In addition, in the syringe main body 6 shown in Fig. 1, no particular additional explosive component is provided, but in order to adjust transition of the pressure applied to the DNA solution via the piston 5, a gas generating agent that generates a gas and the like by combustion of a combustion product generated by explosive combustion in the igniter 71 can be provided in the igniter 71 or in a through-hole of the syringe main body 6. A configuration in which a gas generating agent is provided in the igniter 71 is an already known technique as disclosed in WO 01-031282, Japanese Patent Application Publication No. 2003-25950, and the like. In addition, regarding an example of a gas generating agent, a single base smokeless explosive including 98 mass% of nitrocellulose, 0.8 mass% of diphenylamine, and 1.2 mass% of potassium sulfate may be exemplified. In addition, various gas generating agents used in a gas generator for an airbag and a gas generator for a seat belt pretensioner can be used. When the dimensions, the size, the shape, and particularly, the surface shape of the gas generating agent when provided in the through-hole is adjusted, it is possible to change a combustion completion time of the gas generating agent, and thus the transition of the pressure applied to the DNA solution can be a desired transition, that is, a transition in which the DNA solution can be appropriately injected into the injection target. In the first aspect of the present invention, the driving unit 7 includes a gas generating agent and the like used as necessary.

### (Injection target)

The injection target in the first aspect of the present invention has no limitation, and may be, for example, any of cells, cells in cell sheets, cells in tissues, cells in organs (body organs), cells in organ systems, and cells in individuals (living bodies). Examples of a preferable injection target include injection targets derived from mammals. The injection target is more preferably cells in a mammalian individual (living body), still more preferably cells in the skin, and yet more preferably cells in one or more tissues selected from the group consisting of intradermal, subcutaneous and cutaneous muscles.

Here, when the mammalian individual (living body) has an adipose layer, the subcutaneous muscle includes the adipose layer. For example, swine have a thick adipose layer, whereas rats do not have an adipose layer or if they have an adipose layer, the adipose layer is thin.

In this case, a method in which a solution containing biomolecules is injected from an injector into a skin surface of a mammalian individual (living body), and injected from the skin surface into cells in one or more tissues selected from the group consisting of intradermal, subcutaneous and cutaneous muscles in the skin can be used.

In addition, a system in which a solution containing biomolecules is injected from an injector into an injection target may be any of an in vitro system, an in vivo system, and an ex vivo system.

In addition, the mammal is not particularly limited, and examples thereof include humans, mice, rats, guinea pigs, hamsters, cows, goats, sheep, swine, monkeys, dogs, and cats. In addition, depending on the injection target, a form in which humans are excluded from mammals may be exemplified.

### (Method of confirming that solution containing biomolecules is directly injected into cell nucleus of injection target)

A method of confirming that a solution containing biomolecules is directly injected into a cell nucleus of an injection target is not particularly limited, and a known biological technique can be used. For example, a method in which biomolecules are fluorescently labeled in advance, injected into a cell nucleus of an injection target, and then observed under a fluorescence microscope may be exemplified. In examples to be described below, a Cy3-labeled plasmid V7905 (commercially available from Mirus Bio LLC.) is used as DNA that is directly injected into a cell nucleus of cells in the mammalian individual (living body) and DAPI is used as a nuclear staining dye. For example, a sample can be prepared by acquiring a tissue immediately after injecting DNA and separating it into pieces. In this case, DAPI staining may be performed simultaneously. Red fluorescence is exhibited at a position at which the Cy3-labeled plasmid V7905 is injected, and blue fluorescence is exhibited due to DAPI at a position of the cell nucleus. Therefore, according to observation under a fluorescence microscope, a position at which blue purple fluorescence is exhibited can be identified as a position of the Cy3-labeled plasmid V7905 directly injected into the cell nucleus.

### <Second aspect>

The second aspect of the present invention is a method of injecting a solution containing biomolecules to a cell nucleus of an injection target using the injector of the first aspect.

The description of the first aspect of the present invention above applies to the injector, the injection target, and the solution containing biomolecules in the second aspect of the present invention.

### EXAMPLES

Hereinafter, the present invention will be described in more detail with reference to examples, but the present invention is not limited to the following examples without departing from the spirit and scope of the invention.

### (Evaluation of injection speed of injector)

### [Example 1-1]

The injector shown in Fig. 1 (nozzle diameter: diameter of 0.1 mm) was filled with 100 µL of water, and the injection speed of water from the injection start time of water due to combustion of an ignition charge was evaluated. Regarding the explosive, 35 mg of an explosive containing zirconium and potassium perchlorate (ZPP) was used, and no gas generating agent was used. The injection speed of water was obtained by imaging a distal end of an injector using a high-speed camera (FASTCAM SA-X2 commercially available from PHOTRON LIMITED) and calculating the displacement for injected water and time.

### [Example 1-2]

This example was the same as Example 1-1 except that 15 mg of ZPP was used.

### [Example 1-3]

This example was the same as Example 1-1 except that 55 mg of ZPP was used.

### [Example 1-4]

This example was the same as Example 1-1 except that 90 mg of ZPP was used.

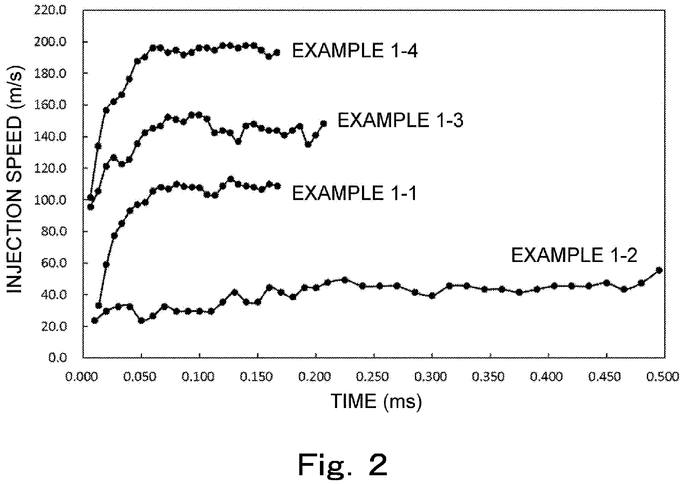

Fig. 2 and Table 1 are a graph and a table showing the injection speed of water in examples over time. Here, in Table 1, the injection speed at a certain time was obtained by dividing a difference between the displacement of water at a time one time before the time and the displacement of water at a time one time after the time by the time. For example, in Example 1-1, the injection speed at a time of 0.013 ms column was obtained by dividing a difference between the displacement of water at a time of 0.000 ms and the displacement of water at a time of 0.020 ms by the time of 0.020 ms.

**[Table 1]**

| Example 1-1 | | Example 1-2 | | Example 1-3 | | Example 1-4 | |
|---|---|---|---|---|---|---|---|
| Time (ms) | Injection speed (m/s) | Time (ms) | Injection speed (m/s) | Time (ms) | Injection speed (m/s) | Time (ms) | Injection speed (m/s) |
| 0.000 | | 0.000 | | 0.000 | | 0.000 | |
| 0.013 | 33.3 | 0.010 | 23.7 | 0.006 | 95.8 | 0.006 | 101.4 |
| 0.020 | 59.2 | 0.020 | 29.7 | 0.013 | 105.6 | 0.013 | 133.9 |
| 0.027 | 77.3 | 0.030 | 32.6 | 0.020 | 121.2 | 0.020 | 156.6 |
| 0.033 | 85.0 | 0.040 | 32.6 | 0.026 | 126.9 | 0.026 | 162.3 |
| 0.040 | 93.2 | 0.050 | 23.7 | 0.033 | 122.7 | 0.033 | 166.5 |
| 0.047 | 97.2 | 0.060 | 26.7 | 0.040 | 125.5 | 0.040 | 176.3 |
| 0.053 | 98.7 | 0.070 | 32.6 | 0.046 | 135.4 | 0.046 | 187.7 |
| 0.060 | 105.7 | 0.080 | 29.7 | 0.053 | 142.5 | 0.053 | 190.5 |
| 0.067 | 108.0 | 0.090 | 29.7 | 0.060 | 145.3 | 0.060 | 196.2 |
| 0.073 | 106.8 | 0.100 | 29.7 | 0.066 | 146.8 | 0.066 | 196.2 |
| 0.080 | 109.8 | 0.110 | 29.7 | 0.073 | 152.4 | 0.073 | 193.3 |
| 0.087 | 108.3 | 0.120 | 35.6 | 0.080 | 151.0 | 0.080 | 194.7 |
| 0.093 | 108.0 | 0.130 | 41.5 | 0.086 | 149.5 | 0.086 | 191.9 |
| 0.100 | 107.6 | 0.140 | 35.6 | 0.093 | 153.8 | 0.093 | 193.4 |
| 0.107 | 103.5 | 0.150 | 35.6 | 0.100 | 153.8 | 0.100 | 196.3 |
| 0.113 | 103.2 | 0.160 | 44.5 | 0.106 | 151.1 | 0.106 | 196.2 |
| 0.120 | 108.7 | 0.170 | 41.5 | 0.113 | 142.5 | 0.113 | 194.8 |
| 0.127 | 113.1 | 0.180 | 38.5 | 0.120 | 143.9 | 0.120 | 197.6 |
| 0.133 | 109.8 | 0.190 | 44.5 | 0.126 | 142.5 | 0.126 | 197.6 |
| 0.140 | 108.7 | 0.200 | 44.5 | 0.133 | 136.9 | 0.133 | 196.2 |
| 0.147 | 108.0 | 0.210 | 47.8 | 0.140 | 146.7 | 0.140 | 197.6 |
| 0.153 | 106.5 | 0.225 | 49.4 | 0.146 | 148.1 | 0.146 | 197.6 |
| 0.160 | 109.8 | 0.240 | 45.5 | 0.153 | 145.3 | 0.153 | 194.7 |
| 0.167 | 108.7 | 0.255 | 45.5 | 0.160 | 143.9 | 0.160 | 190.5 |
| | | 0.270 | 45.5 | 0.166 | 143.9 | 0.166 | 193.4 |
| | | 0.285 | 41.5 | 0.173 | 141.1 | | |
| | | 0.300 | 39.5 | 0.180 | 143.9 | | |
| | | 0.315 | 45.5 | 0.186 | 146.7 | | |
| | | 0.330 | 45.5 | 0.193 | 135.3 | | |
| | | 0.345 | 43.5 | 0.200 | 141.0 | | |
| | | 0.360 | 43.5 | 0.206 | 148.2 | | |
| | | 0.375 | 41.5 | | | | |
| | | 0.390 | 43.5 | | | | |
| | | 0.405 | 45.5 | | | | |
| | | 0.420 | 45.5 | | | | |
| | | 0.435 | 45.5 | | | | |
| | | 0.450 | 47.4 | | | | |
| | | 0.465 | 43.5 | | | | |
| | | 0.480 | 47.4 | | | | |
| | | 0.495 | 55.3 | | | | |

### (Test of injecting DNA solution into cell nucleus of cells in mammalian individual (living body))

### [Example 2-1]

The injector used in Example 1 was filled with 30 µL of a solution containing a Cy3-labeled plasmid V7905 (solvent: endotoxin-free TE buffer, final concentration: 0.1 mg/mL). Regarding the explosive, 35 mg of an explosive containing zirconium and potassium perchlorate (ZPP) was used, and regarding the gas generating agent, 40 mg of a single base smokeless explosive. The solution was injected into the skin of the lumbar back of a female SD rat (10-week old). Here, as described above, no gas generating agent was used in Example 1-1, but the gas generating agent was used in this example. This is because it is considered that use of a gas generating agent does not affect the initial injection speed defined in the present invention.

Immediately after injection, the skin was removed and frozen in an OCT compound (embedding agent for preparing a frozen tissue section (Tissue Tech O.C.T. Compound), commercially available from Sakura Finetek Japan Co., Ltd.) with dry ice. Using a cryostat (commercially available from Leica), the cross section of the injection part was cut into slices with a thickness of 6 µm and encapsulated with an encapsulant containing DAPI. The fluorescence of the prepared sample was observed under an all-in-one fluorescence microscope (Z-X700, commercially available from Keyence Corporation), and a red fluorescence image of Cy3 and a blue fluorescence image of DAPI were obtained with a thickness of 0.1 to 0.4 µm. In order to obtain an injection distribution in the injection area, images in a plurality of fields were obtained. The results are shown in Fig. 3-1. The scale bar indicates 0.73 mm.

A proportion of the number of cells into which DNA was directly injected was calculated as follows using a hybrid cell count function. That is, for cells in each analysis target area (each area surrounded by a white frame in Fig. 3-1), cells in which an area of the purple fluorescence in which the blue fluorescence and the red fluorescence overlapped was 50% or more with respect to the area of cells were defined as cells into which DNA was directly injected, and the number of cells was counted (this is referred to as a number of cells A). On the other hand, a total number of cells in each analysis target area was counted using the number of cell nuclei as an index (this is referred to as a number of cells B). A ratio shown in each analysis target area in Fig. 3-1 is a ratio of the number of cells A to the number of cells B. Here, the epidermis and hair follicles in which hardly any of the red fluorescence of Cy3 was observed were excluded from the analysis target.

### [Example 2-2]

This example was the same as Example 2-1 except that 15 mg of ZPP was used. The results are shown in Fig. 3-2.

In Fig. 3-1 and Fig. 3-2, in Example 2-1 and Example 2-2, DNA was directly injected into the cell nucleus in intradermal cells. In addition, it was found that the proportion of DNA directly injected into these cell nuclei was significantly large also in cells present in the injection target in the linear direction from the injection port.

### [Example 2-3]

This example was the same as Example 2-1 except that 55 mg of ZPP was used, and female swine (15-week old, edible Sangenton (LWD) (hybrid of Landrace species, Yorkshire species, and Duroc species)) were used in place of the female SD rat (10-week old), and injection into the skin of the abdomen was performed.

Here, when a proportion of the number of cells into which DNA was directly injected was calculated, as analysis target areas, an area (a1) surrounded by a white dashed frame on the upper side and an area (b1) surrounded by a white dashed frame on the lower side in Fig. 3-3 were used. The area (a1) was a part of the intradermal cells and the area (b1) was a part of the adipose layer.

As shown in Fig. 3-5A, an image (a2) including a numerical value was obtained by dividing the area (a1) into a plurality of sections, and the ratio of the number of cells A to the number of cells B, the number of cells B, and the number of cells A were added to each of the sections. For example, the description "2.4 (2/83)" indicates that the ratio of the number of cells A to the number of cells B was 2.4%, the number of cells B was 2, and the number of cells A was 83. A section in which no numerical value is shown is a section in which the ratio of the number of cells A to the number of cells B was 0.

The same applies to the image (b2) shown in Fig. 3-5B.

### [Example 2-4]

This example was the same as Example 2-3 except that 90 mg of ZPP was used. The results are shown in Fig. 3-4, Fig. 3-6A and Fig. 3-6B.

In Fig. 3-3 to Fig. 3-6B, in Example 2-3 and Example 2-4, it was possible to directly inject DNA into the cell nucleus of intradermal cells and the cell nucleus of subcutaneous cells, and directly inject DNA into the cell nucleus of cells in the adipose layer subcutaneously.

### DESCRIPTION OF REFERENCE NUMERALS

1: Syringe, 2: Housing, 3: Syringe part, 4: Plunger, 5: Piston, 6: Syringe main body, 7: Driving unit, 8: Button, 9: Battery, 10: Syringe assembly, 31: Nozzle unit, 31a: Injection port, 32: Filling chamber, 71: Igniter

## Claims

1. An injector that injects a solution containing biomolecules into an injection target from an injector main body without performing injection through a given structure in a state where the given structure is inserted into the injection target, the injector comprising:
an accommodation unit for accommodating a solution containing biomolecules; and
a nozzle unit including an injection port through which the solution containing biomolecules flows and is injected into the injection target, the solution being pressurized,
wherein there is a time at which an injection speed of the solution containing biomolecules is 40 m/s or more between an injection start time of the solution containing biomolecules and a time of 0.20 ms.

2. The injector according to claim 1,
wherein there is a time at which the injection speed of the solution containing biomolecules is 75 m/s or more between the injection start time of the solution containing biomolecules and a time of 0.20 ms.

3. The injector according to claim 1 or 2,
wherein there is a time at which the injection speed of the solution containing biomolecules is 75 m/s or more between the injection start time of the solution containing biomolecules and a time of 0.15 ms.

4. A method of injecting a solution containing biomolecules into a cell nucleus of an injection target using the injector according to any one of claims 1 to 3.
